Europäisches Patentamt

European Patent Office

Office européen des brevets

(19)

(11) Publication number: **0 217 061 B1**

(12) **EUROPEAN PATENT SPECIFICATION**

(45) Date of publication of patent specification: **26.07.95**

(51) Int. Cl.⁶: **C07K 1/14**, A61K 35/16, G01N 33/577

(21) Application number: **86110672.2**

(22) Date of filing: **14.12.82**

(60) Publication number of the earlier application in accordance with Art.76 EPC: **0 083 483**

(54) **Ultrapurification of factor VIII.**

(30) Priority: **14.12.81 US 330105**

(43) Date of publication of application:
**08.04.87 Bulletin 87/15**

(45) Publication of the grant of the patent:
**26.07.95 Bulletin 95/30**

(84) Designated Contracting States:
**BE CH DE FR GB IT LI LU NL SE**

(56) References cited:

**CHEMICAL ABSTRACTS, vol. 90, no. 16, 16th April 1979, page 367, right-handcolumn, abstract no. 127439p, Columbus, Ohio, US; E.G.D. TUDDENHAM et al.: "Theproperties of factor VIII coagulant activity prepared by immunoadsorbentchromatography", & J. LAB. CLIN. MED. 1979, 93(1), 40-53**

(73) Proprietor: **SCRIPPS CLINIC AND RESEARCH FOUNDATION**
**10666 North Torrey Pines Road**
**La Jolla**
**California 92037 (US)**

(72) Inventor: **Zimmerman, Theodore Samuel**
**544 Bon Air Street**
**La Jolla**
**California 92037 (US)**
Inventor: **Fulcher, Carol Ann**
**7543 La Jolla Boulevard**
**La Jolla**
**California 92037 (US)**

(74) Representative: **Ablewhite, Alan James et al**
**MARKS & CLERK,**
**57/60 Lincoln's Inn Fields**
**London WC2A 3LS (GB)**

EP 0 217 061 B1

CHEMICAL ABSTRACTS, vol. 96, no. 25, 21st June 1982, page 366, left-handcolumn, abstract no. 213755u, Columbus, Ohio, US; G.J. KNUTSON et al.: "Porcinefactor VIII:C prepared by affinity interaction with von Willebrand factor andheterologous antibodies: sodium dodecyl sulfate polyacrylamide gel analysis", &

BLOOD 1982, 59(3), 615-624

CHEMICAL ABSTRACTS, vol. 89, no. 7, 14th August 1978, page 248, left-handcolumn, abstract no. 55806k, Columbus, Ohio, US; L. HOLMBERG et al.:"Purification of F.VIII:C by antigen-antibody chromatography", & THROMB. RES.1978, 12(4) 667-675

**Description**

This invention relates generally to a method of separating and purifying factor VIII procoagulant activity protein. More specifically, high purity factor VIII procoagulant activity protein is separated from von Willebrand Factor by a two step chromatographic adsorption and concentration technique from plasma or concentrate.

The isolation of the antihemophilic factor from blood plasma has been described in the literature. The precise structure of the antihemophilic factor, also referred to as factor VIII, has not yet been identified, due in part, to the unavailability of sufficient quantities of pure material with which to conduct further studies. The limited availability of pure material and its existence in a dilute state has also hindered its use in therapeutic applications.

Factor VIII procoagulant activity protein functions to correct the clotting defect in hemophilic plasma. It circulates in plasma complexed with the von Willebrand factor protein. The latter can alter the platelet function defect in von Willebrand's disease. That portion of the factor VIII/von Willebrand factor complex having coagulant activity is referred to as factor VIII procoagulant activity protein, factor VIII - clotting activity or simply VIII:C (the designation of "VIII:C" will be used herein after to identify the portion of the factor VIII molecule with such clotting activity.) The other portion of the factor VIII/von Willebrand factor complex having the ability to correct the platelet function defect in von Willebrand's disease is referred to as von Willebrand factor, factor VIII - related antigen, VIIIR:Ag,VIII:RP factor. (The description "VIII:RP" will be used hereinafter to identify the platelet correction function of the factor VIII molecule). Although yet unproven, there is evidence to support the conclusion that VIII:C exhibits properties and the behavior of a small molecule which is combined with VIII:RP as a non-covalent complex. There is also a basis for the contention that the properties associated with both VIII:C and VIII:RP may also be a single molecule which under appropriate conditions may be cleaved, yielding two fragments.

In view of the need for identifying the structures of the factor VIII/von Willebrand factor complex, VIII:C and VIII:RP and the important pharmaceutical value of the coagulant activity ascribable to VIII:C, numerous attempts have been made to purify factor VIII and to separate and concentrate VIII:C and VIII:RP. The techniques used are based generally on either immunaodsorption or ion exchange chromatography. Such techniques as heretofore used have had limited success due to the difficulty of desorbing the proteins from the charged ionic material in an undamaged condition or recovering same in suitable quantities.

One such method for separating VIII:C from VIII:RP utilizing immunoadsorbent chromatography has been reported by E.G.D.Tuddenham et al, "The Properties of Factor VIII Coagulant Activity Prepared by Immunoadsorbent Chromatography", JOURNAL OF LABORATORY CLINICAL MEDICINE, Vol. 93, p. 40 (1979). The reported method is a one-step separation of VIII:C from nearly all VIII:RP and from most other plasma proteins employing a chromatographic column packed with agarose beads to which polyclonal antisera to VIII:RP (anti-VIII:RP) are coupled. Factor VIII/von Willebrand factor containing plasma is passed through the column which adsorbs both VIII:C and VIII:RP. Other unwanted plasma proteins are removed from the column by washing with buffered saline solution and the desired VIII:C is obtained by subsequent elution with a calcium-ion gradient. Although it is stated to be an improvement in both purity and yield of VIII:C, when compared to the previously known methods, it is also stated that the resulting product also contains VIII:RP and other plasma proteins. Such contaminants may be attributable to the use of polyclonal antisera bound to the agarose beads. Since a majority of the immunoglobul ins from which the antisera are constituted are not specific to VIII:RP, the effective number of sites where antibodies specific to VIII:RP may be bound to agarose is relatively small due to competition between the antisera for a finite number of bonding sites on the agarose.

Another method for separating VIII:C from VIII:RP and ristocetin co-factor by a chromatographic technique employing aminohexyl-substituted agarose has been described by D.E.G Austen, "The Chromatographic Separation of Factor VIII on Amino-hexyl Sepharose," BRITISH JOURNAL OF HAEMATOLOGY, Vol. 43, p. 669 (1979). The described method is stated to be an improved method for the component parts of both human and porcine factor VIII/von Willebrand factor. This method, however, also suffers from the fact that contaminants are present in the resulting product. In both the Tuddenham et al and Austen methods a contaminated product which is more dilute than is normally desired, is formed.

Hence, it is clear that there still exists a need for an improved method for separating and purifying VIII:C from VIII:RP using plasma or concentrates. Therefore, it is an object of the present invention to satisfy such a need.

The present invention relates to a method of separation of the component molecules of the factor VIII/von Willebrand factor complex, VIII:C and VIII:RP, and the purification and concentration of the procoagulant activity protein VIII:C. This method achieves the object of producing highly purified VIII:C using a

3

EP 0 217 061 B1

two step procedure. According to the invention there is provided a method of preparing human Factor VIII procoagulant activity protein comprising the steps of

(a) adsorbing a VIII:C/VIII:RP complex from a plasma or commercial source onto a solid substrate to which is bound a monoclonal antibody which is specific to VIII:RP and which remains complexed to VIII:RP when subjected to a saline solution and when bound to a substrate, the monoclonal antibody being capable of achieving, in step (d) below, a VIII:C product with a fold purification greater than 160,000, relative to plasma;

(b) eluting the VIII:C;

(c) concentrating the VIII:C obtained in step (b) by affinity chromatography;

(d) recovering highly purified and concentrated VIII:C.

The first step involves immunoadsorption of factor VIII from plasma or a commercial concentrate. The adsorbent employed comprises a monoclonal antibody specific to VIII:RP which is bound to be a suitable substrate, such as agarose beads. After the VIII:C/VIII:RP is initially adsorbed, the substrate particles are washed extensively with a buffer solution to remove unadsorbed protein. The adsorbed material is then treated with a calcium ion containing solution to elute the adsorbed VIII:C. The VIII:RP portion remains adsorbed on the anti-VIII:RP bound material. At this point about 40-60% of the VIII:C initially adsorbed is recovered in a highly purified state. However, the procoagulant activity protein recovered, although extremely pure, i.e., largely free from contaminants, is too dilute to be of significant therapeutic value.

The second step of the present process is directed to substantially concentrating the recov ered purified VIII:C using a technique which may be characterized as affinity chromatography.

The VIII:C solution obtained from the first step of the present process having a potency of approximately 10-20 International Units (hereinafter "units") is processed in a column containing aminohexyl substituted agarose. The column is then washed with a buffer solution and the VIII:C is eluted with a calcium ion-containing solution to yield a VIII:C concentration in excess of 1000 units per ml, and being greater than 160,000 fold purified from plasma. Thus, the present method yields unexpectedly high purity procoagulant activity protein in a highly concentrated and therapeutically useful state. Methods used heretofore fail to achieve such notable results for several reasons. The method of Tuddenham et al, described earlier, employs bound polyclonal antisera instead of the specific and highly selective monoclonal antibodies to VIII:RP as used in the present invention. As a result, fewer specific antibodies to VII:RP are coupled for a given weight of agarose. In the method of the present invention monoclonal antibodies are exclusively bound to a relatively inert substrate. When the method of Tuddenham et al is used only 2.6 to 6.4 units of VIII:RP per ml of immunoglobulin-agarose beads (equivalent to 53.1-82.9% of the amount applied to the column) are removed. This compares to greater than 1000 units per ml of beads (or 90-100% of the VIII:RP which is applied to the column) which is recovered when the monoclonal antibody immunoadsorbent of the present invention is employed. This ability to adsorb more VIII:C/VIII:RP (factor VIII/von Willebrand factor) per ml of beads accordingly results in a higher concentration of VIII:C when it is subsequently eluted from the immunoadsorbent. Thus, 10-20 units of VIII:C per ml of eluant are obtained with the present invention, in contrast to 0.5-1.25 units per ml of eluant with the Tuddenham et al method.

The present method also permits the selection of a monoclonal antibody having a high affinity for VIII:RP; however, the use of polyclonal antibodies results in varying affinities. It should be realized that there is an inverse relationship between the affinity of the bound antibody for VIII:RP and the elution of VIII:RP. Thus, the higher the affinity of the antibody for VIII:RP, the less VIII:RP will be present with VIII:C in the eluant. The present invention also makes it possible to produce an unlimited supply of the specified monoclonal antibody, thus eliminating variations among different batches.

According to a further feature of the invention there is provided an improved immunoadsorbent for isolation and purification of human VIII:C from VIII:C/VIII:RP comprising a monoclonal antibody specific to VIII:RP, which remains complexed to VIII:RP when subjected to a saline solution and when bound to a substrate and being capable of achieving a highly purified factor VIII:C with a fold purification greater than 160,000 relative to plasma, said monoclonal antibody being bound to solid resin particles.

Although Austen, as earlier described, has reported the use of aminohexyl-agarose to separate VIII:C from VIII:RP, such a material has not heretofore been used to concentrate VIII:C following a separation and purification step. Heretofore, the highest VIII:C concentrations achieved by using aminohexyl agarose in chromatography were 0.53 units per ml of eluant for human protein and 2.38 per ml of eluant for porcine VIII:C. The present method permits concentrations several orders of magnitude greater than these. Perhaps of even greater significance, is the fact that the present invention provides a greater purification of human VIII:C than has even been reported (164,000 vs 17,000 fold over plasma). The present method, which is described in more detail hereinafter, yields VIII:C with a specific activity of about 2,300 units/mg when commercial concentrate is used. This corresponds to a 164,000 fold purification from plasma. The ratio of

4

VIII:C to VIII:RP is greater than $10^5$ as compared to the ratio in plasma.

The following description provides, by way of example only, details of the manner in which typical embodiments of the present invention may be made and used in order to achieve the separation, purification and concentration of VII:C to a degree of purity and concentration not known heretofore.

### A. Preparation of Monoclonal Antibody to VIII:RP

The monoclonal antibody to VIII:RP which is subsequently bound to the separation substrate may be prepared in a stepwise procedure starting with a highly purified preparation of factor VIII/von Willebrand factor (VIII:C/VIII:RP complex). The purification is accomplished with material obtained from a plasma source or less highly purified material is used in higher concentration such as commercial extracts available as FACTORATE (trademark of Armour Pharmaceutical Co., Tuckahoe, N.Y.) or Hemophil (trademark of Hyland Laboratories, Costa Mesa, California). Purification is performed by a standard agarose-gel filtration of cryoprecipitate, such as that described by Zimmerman and Roberts, "Factor VIII Related Antigen", appearing in IMMUNOASSAYS: CLINICAL LABORATORY TECHNIQUES FOR THE 1980's, R.M.Nakamura et al, eds., Alan R. Liss, Inc., New York, pp. 339-349 (1980). Mice were injected with highly purified factor VIII/von Willebrand factor obtained from plasma according to the following procedure. On day zero, the mice are injected intraperitoneally with a composition prepared by dissolving (or suspending) 10 mg of the protein in 0.1 ml of buffer containing 0.05 M Tris, 0.15 M sodium chloride, 0.02% sodium azide, 1 mM phenyl methyl sulfonyl fluoride, trasylol 10 units/ml at pH7.3. and shaking with an equal volume of complete Freund's adjuvant. On day 14, the mice are again injected with the same material except that incomplete Freund's adjuvant is substituted for complete Freund's adjuvant. On day 21, the injection of day 14 is repeated. On day 38, the mice are injected with purified VIII:C/VIII:RP only. On day 42, the spleens of the mice are removed and fused according to a standard procedure, of the type described by J.P.Brown et al "Protein Antigens of Normal and Malignant Human Cells Identified by Immunoprecipitation with Monoclonal Antibodies", JOURNAL OF BIOLOGICAL CHEMISTRY, Vol. 225, pp. 4980-4983 (1980). The standard technique is varied only to the extent that 35% polyethylene glycol 1000 is substituted for 50% polyethylene glycol. A radioimmunoassay method for clones producing antibody to VIII:RP is performed according to the following procedure. Polyvinyl plates with a "V" bottom, flexible type are coated with 0.1 ml of factor VIII purified from commercial extract according to the procedure indicated above and having a concentration of 0.125 mg/ml of protein. The plates are then washed and reacted with rabbit anti-mouse IgG antiserum, washed a second time and $^{125}$I labeled goat anti-rabbit IgG antiserum is added to the wells and incubated.

The plates are blocked with albumin, washed with buffer and incubated with the culture fluids from the clones to be tested. The plates are again washed, then dried and the wells cut-out and counted. After determining the clones which are positive they are subcloned at least twice and stable clones producing antibody to VIII:RP are then injected into the peritoneal cavities of Balb/C mice which have been pretreated intraperitoneally with 0.5 ml of pristane at least four days prior to injection of cells. Hybridoma cells are injected at concentrations of approximately $5 \times 10^6$ cells per mouse in 0.5 ml of Delbecco's modified Eagle's medium without fetal bovine serum. The mice are tapped when bloated and ascites fluid is collected in heparin at approximately 10 units/ml. As cites fluid from multiple mice is pooled to provide a convenient volume for subsequent isolation of the monoclonal IgG. If the heparinized ascites fluid is not used immediately, it may be stored at -70°C and thawed just prior to use. The final yield of IgG from the ascites fluid is approximately 1g of IgG per 100ml of ascites fluid.

The specificity of the monoclonal IgG for the purpose of purifying VIII:C may be assessed by coupling the IgG to a separation substrate medium, in the manner described hereinafter, and demonstrating that the bound IgG removes both VIII:RP and VIII:C from plasma and that the VIII:C may be subsequently eluted with a solution containing calcium ions while the VIII:RP remains complexed to the monclonal IgG which is bound to the solid-state substrate.

The monoclonal IgG, which is to be used subsequently to prepare the immunoadsorbent, may be isolated from heparinized pooled ascites fluid immediately after collection or a frozen portion of the stored solution may be thawed. Regardless of whether fresh or frozen material is used, the solution is brought to 4°C and treated with an equal volume of phosphate buffered saline solution (PBS), the composition of which is set forth below. The diluted ascites is precipitated by dropwise addition with stirring at 4°C of an equal volume of saturated ammonium sulfate (SAS); prepared by boiling an excess of ammonium sulfate in water, cooling to 4°C, filtering undissolved crystals and adjusting the pH to 7.0 with ammonium hydroxide. The precipitate and its supernatant liquid are stirred for at least 2 hours and centrifuged at 4°C. Centrifugations are preferably carried out at 14,000 rpm for 60 minutes (30,000 x g). The supernatant solution of ascites is precipitated twice more with SAS and the mixture of precipitate and supernatant liquid stirred and centrifuged in the same manner as in the first cycle. The pellets resulting from the third precipitation are resuspended in a volumn of PBS equal to that of the diluted ascites fluid and then dialyzed exhaustively against PBS. Clots appearing in the dialysis bags are removed by centrifugation at 20°C. The dialyzed IgG is adsorbed by stirring it with a 5% aqueous solution of aluminum hydroxide at room temperature and centrifuging at 20°C after adsorption. The adsorption treatment is repeated at least three more times using 2.5% aluminum hydroxide solution for each treatment after the first. The adsorbed IgG is brought to 4°C and reprecipitated once with SAS as described above. The precipitated pellets may be stored at -20°C until used.

B. Preparation of Immunoadsorbent

The immunoadsorbent is prepared by suitably preparing the monoclonal IgG for coupling, preparing the solid substrate for coupling and reacting the two components to bind the former to the later.

(i) Preparation of IgG for Coupling

Either freshly precipitated IgG may be used or previously frozen precipitate may be thawed for use. The material is then dialyzed against PBS, and while still in the PBS, the volume and IgG concentration ($A_{280}/1.4$ = mg/ml IgG) are determined. The IgG is then treated with between 10 and 30 microliters, preferably 20 microliters, of diisopropylfluorophosphate per 50 ml of IgG solution. The resulting solution is stirred at room temperature in a hood for 30 minutes and the treated IgG, immediately prior to use, is dialyzed overnight against coupling buffer. The coupling buffer found most suitable is a 0.25M sodium bicarbonate solution adjusted to a pH of 9, preferably with sodium hydroxide.

(ii) Preparation of Solid Substrate for Coupling

Although the monoclonal antibody may be bound to any material which does not have a high affinity for protein, particularly factor VIII itself, such materials as glass beads, agarose and derivatives thereof are preferred. Most preferred is a cross-linked agarose available commercially as a gel known as Sepharose CL2B (trademark of Pharmacia Fine Chemicals, Piscataway, N.J.).

The method of preparing the preferred immunoadsorbent resin is generally the same as that disclosed in the literature, such as the method of J. Porath et al, JOURNAL OF CHROMATOGRAPHY, Vol. 86, pp. 53-56 (1973). The method found most suitable is as follows: a volume of about 2 liters of Sepharose CL2B is placed in an acid-cleaned 2 liter sintered glass filter funnel. The resin is washed with water and filtered to a moist cake. The washed resin is placed in a large (approximately 4 liter) glass beaker equipped with a magnetic stirring bar. To the resin is then added 750 ml of cold potassium phosphate buffer solution, prepared by mixing one part of a 5M dibasic potassium phoshpate solution with two parts of 5M tribasic potassium phosphate solution. Sufficient cold water is added to bring the final volume to 3 liters. The mixture is then chilled to 4°C and maintained at between 4-10°C in an ice-water bath placed on a magnetic stirring plate. In a hood, cyanogen bromide is added to 30.0 ml of water in a stoppered glass bottle containing a magnetic stirring bar. The mixture is rapidly stirred until solution results. The cyanogen bromide solution is then added with stirring over a 2 minute period to the cold Sepharose mixture. Stirring is continued for an additional 8 minutes and then transferred to a chilled 2 liter sintered glass filter funnel supported in a 4 liter vacuum flask. The cyanogen bromide treated resin is then washed with approximately 20 liters of cold water or until the pH of the filtrate is neutral. The washed resin is then quickly equilibrated with cold coupling buffer and then transferred to a 4 liter plastic beaker equipped with a large magnetic stirring bar.

(iii) Coupling the Monoclonal Antibody to the Solid Substrate

The solid substrate resin, prepared as indicated above, is ready to be used when it is equibrated with coupling buffer and should not be stored thereafter. Accordingly, the resin mixture is combined with the IgG which was previously dialyzed overnight against coupling buffer. The combined resin IgG suspended mixture is stirred at 4°C for a period of about 24 hours. The $A_{280}$ of an undiluted sample of the supernatant coupling liquid may be determined using bovine serum albumin (BSA) as a standard or Bio-Rad protein assay (Bradford reagent) with BSA as standard. The percentage ligand which is coupled may then be calculated. When the above described procedure is followed, this is usually about 95%. Any remaining active sites on the resin not coupled to antibody may be blocked by washing the resin on a sintered glass

EP 0 217 061 B1

filter funnel with cold coupling buffer containing 0.1M glycine. The resin is then resuspended in this solution to a final volume equal to that when the resin and antibody, each in coupling buffer, were combined. The suspension is stirred slowly overnight at 4°C. The resin is then washed thoroughly with VIII:C-buffer, the composition of which is given below. The coupled, blocked resin is then pre-eluted with VIII:C-buffer additionally containing 0.5M calcium ions, preferably calcium chloride. The resin is again washed with VIII:C buffer alone and stored at 4°C or in a continuously pumped column at room temperature until ready for use. The coupling density of IgG to SEPHAROSE should be 2-5g, preferably 3-4g IgG/liter of SEPHAROSE.

C. Separation and Purification of VIII:C

(i) Sample preparation of factor VIII, such as human plasmas and commercial concentrates of factor VIII, may be employed in the present invention and the method is not limited as to a particular type of material. Preferred materials, and those which have demonstrated successful results, are human plasmas and commercially available concentrates of human factor VIII, such as FACTORATE available from Armour Pharmaceutical Co. The following description provides details for using commercial human concentrate such as FACTORATE:

FACTORATE is reconstituted by adding 25 ml portions of VIII:C-buffer to the contents of each of 20 bottles (25 ml per bottle). The mixture is adjusted to a final volume of 1 liter with VIII:C-buffer. A sample aliquot of 0.5ml may be removed for assay and the remaining material applied to the immunoadsorbent column overnight at a rate of approximately 60 ml/hour.

It should be noted that while the description of the present invention refers, and is directed primarily, to the use of immunoadsorbent coupled particles in a chromatography column, it is within the scope of this invention to perform batchwise separations by placing the antibody-bound resin particles in a suitable container and after adding reconstituted concentrate or plasma, VIII:C as outlined above and described in more detail below.

When the process is carried out in a chromatography process, the following embodiments are preferred:

The resin is placed in a column, such as Amicon 86001, (trademark of Amicon Corp., Lexington, Mass.), equipped with a peristaltic pump and a high flow head. When concentrate is used as the source of factor VIII, for 20 bottles of diluted concentrate, approximately 1.5 liters of resin, prepared as indicated above, is used. When porcine plasma is used, 150 ml of resin is used for each liter of plasma.

After the sample is applied to the column, it is washed with 1 liter of VIII:C-buffer, followed by a second washing with VIII-C-buffer which additionally contains 0.5M NaCl. Approximately 20 liters of salin-buffer is used when factor VIII is applied as concentrate and 20 bed volumes when porcine plasma is employed. Optimum results are obtained with a flow rate of 1 liter/hour.

Elution of purified VIII:C is accomplished with VIII:C-buffer containing calcium ions. Although a linear gradient, as taught by Tuddenbram et al, supra, works well, it is not required in order to accomplish the object of this invention; a solution having a fixed calcium ion concentration is quite adequate. Thus, when VIII:C derived from concentrate is being eluted, VIII:C-buffer, 0.25 to 0.5M with respect to calcium chloride, preferably 0.35M, is used advantageously as a flow rate of between 450 to 750 ml/hour and preferably 600 ml/hour.

Fractions of 12 ml are collected for VIII:C originating from concentrate. Those fractions containing at least 1.0 unit/ml of VIII:C activity are pooled and the total volume and activity of the pool determined.

The VIII:C pool is initially concentrated to 10-20 ml by a standard procedure such as pressure ultrafiltration.F For this purpose, Amicon stirred cell in which a YM-10 membrane under 50 psi of nitrogen pressure has been found to work well. Slow stirring is continued for 30 minutes after nitrogen pressure is released, and the volume and activty of the concentrated pool are determined. The pool may be stored for a brief period, that is, overnight for example, if a temperature of 4°C is maintained.

It may be noted that the immunoadsorbent column described above may be regenerated by treatment of the column with 2 bed volumes of 3M aqueous sodium thiocyanate solution run at a flow rate of about 0.5-1 liter/hour to elute VIII:RP.

D. Concentration of Purified VIII:C

Although the VIII:C recovered from the separation from VIII:RP by means of the immunoadsorbent column is highly purified, it is still too dilute to be therapeutically useful. Further concentration is accomplished by use of an aminohexyl agarose column which is prepared and used in the following manner:

7

### (i) Preparation and/or Conditioning of a Aminohexyl Agarose Column

Aminohexyl agarose is agarose which has been reacted with 1,6-diaminohexane to yield an agarose resin having a number of 6 carbon atom chains, each of which has a terminal amino group. It may be prepared according to the method described by Austen, supra, or acquired from a commercial supplier. One such material which has been used successfully in the present invention is available under the name of AH-SEPHAROSE 4B (trademark of Pharmacia Fine Chemicals, Piscataway, N.J).

Whether prepared or purchased, the resin should be conditioned prior to use. This may be accomplished as follows, the volumes, amounts and dimensions being adjusted in proportion to the amount of material to be concentrated:

Approximately 1 gram of aminohexyl agarose (AH-SEPHAROSE 4B) is placed in a sintered glass filter funnel and washed with at least 200 ml of 0.5M sodium chloride, while stirring. The resin is then equilibrated with VIII:C-buffer and packed in a column of approximately 0.9 cm diameter. A Bio-Rad Econo-Column with flow adapters has proven quite suitable for the type of use considered here. The bed volume of the packed column is approximately 4 ml.

### (ii) Application to and use of the Aminohexyl Agarose Column

The concentrated pool, prepared as described above, is diluted 1:10 in VIII:C-buffer to a final concentration of 100-200 ml when using the amounts of resin and column size as described in the immediately preceding section. The diluted pool is applied to the column at a flow rate of 200 ml/hour.

The column is then washed with VIII:C-buffer which contains calcium ions, preferably from calcium chloride. The solution should be between 0.01M to 0.03M, preferably 0.025M with respect to calcium ions.

Elution of the concentrated VIII:C is achieved at a flow rate of between 5 to 20 ml/hour, preferably 10 ml/hour with VIII:C-buffer containing a higher concentration of calcium ions than was employed with the preceding washing step. Again, calcium chloride is the preferred source of calcium ions in a concentration of between 0.25 to 0.5M, preferably 0.3M. Fractions of 1 ml volume are collected and assayed as described below. Collected fractions may stored at 4°C or frozen.

Assays may be performed by diluting the fractions with VIII:C-buffer if necessary and further diluting the fraction 1:100 in assay buffer prior to addition to the substrate. A standard partial thromboplastin time assay is employed.

The composition of the buffer solutions is as follows:

#### Phosphate Buffered Saline Solution

1.6g sodium phosphate, monobasic monohydrate
8.4g sodium phosphate, dibasic anhydrous
61.4 sodium chloride
water to 7 liters
pH of buffer is 7.2

#### VIII:C-Buffer

ml 0.02M imidazole
ml 0.15M sodium chloride
ml 0.10M lysine
ml 0.02% sodium azide
pH of buffer is adjusted with concentrated hydrochloric acid to 6.8.

The date listed hereinafter in Table I are representative of that obtained according to the present invention, as described below.

8

## TABLE 1

VIII:C Obtained from FACTORATE Concentrate

as the Source of VIII:C/VIII:RP

| | Volume (ml) | VIIIC (Units/ml)* | VIIIC (Total Units) | Protein (mg/ml) |
|---|---|---|---|---|
| Sample Applied to Immunoadsorbent | 500 | 18.8 | 9400 | 29 |
| Pool Resulting from Immunoadsorbent | 1020 | 4.6 | 4692 | - |
| Pool After Initial Concentration | 20 | 134 | 2680 | - |
| Sum Resulting from Aminohexyl Column | - | - | 1576 | - |
| Aminohexyl Fraction #3 | 0.95 | 1172 | 1112 | 0.51 |
| Aminohexyl Fraction #4 | - | 545 | - | 0.23 |

*A frozen human plasma pool used as the standard for VIIIC

assays and assigned the value of 1 human unit per ml.

## TABLE 1 cont'd

VIII:C Obtained from FACTORATE Concentrate

as the Source of VIII:C/VIII:RP

| Protein (Total mg) | Recovery (%) | Specific Activity (Units/ mg) | From Plasma (Fold Purif.) |
|---|---|---|---|
| 14,500 | - | 0.7 | 50 |
| - | 50 | - | - |
| - | 29 (57) | - | - |
| - | 17 (59) | - | - |
| 0.48 | 12 | 2294 | 163,857 |
| - | - | 2370 | 169,285 |

* A frozen human plasma pool used as the standard for VIIIC assays and assigned the value of 1 human unit per ml.

## Claims

1. A method of preparing human Factor VIII procoagulant activity protein comprising the steps of
   (a) adsorbing a VIII:C/VIII:RP complex from a plasma or commercial source onto a solid substrate to which is bound a monoclonal antibody which is specific to VIII:RP and which remains complexed to VIII:RP when subjected to a saline solution while bound to the substrate, the monoclonal antibody being capable of achieving, in step (d) below, a VIII:C product with a fold purification greater than 160,000, relative to plasma;
   (b) eluting the VIII:C;
   (c) concentrating the VIII:C obtained in step (b) by affinity chromatography;
   (d) recovering highly purified and concentrated VIII:C.

2. A method according to Claim 1, wherein the elutant used in each of steps (b) and (c) is a buffered salt solution.

3. A method according to Claim 2, wherein the salt solution contains calcium chloride.

4. A method according to Claim 3, wherein the concentration of said calcium chloride solution used in steps (b) and (c) ranges from 0.24M to 0.5M.

5. A method according to any of Claims 1 to 4, wherein said substrate in step (a) is in the form of solid substrate particles.

6. A method according to Claim 5, wherein said substrate is agarose.

7. A method according to any of Claims 1 to 6, wherein aminohexyl agarose is employed in step (c) as the adsorbent.

8. A method according to any of Claims 1 to 7, wherein the monoclonal antibody used is an IgG.

9. A method according to Claim 8, wherein the monoclonal antibody used is a mouse IgG.

10. A method for purifying human Factor VIII procoagulant activity protein from plasma or concentrate, including the step of passing said plasma or concentrate through a chromatographic column containing a resin adsorbent to which are bound monoclonal antibodies as defined in Claim 1 and eluting the VIII:C therefrom.

11. A method according to Claim 10, wherein said adsorbent is agarose and said elutant is a solution of a salt.

12. An improved immunoadsorbent for isolation and purification of human VIII:C from VIII:C/VIII:RP comprising a monoclonal antibody specific to VIII:RP, which remains complexed to VIII:RP when subjected to a saline solution while bound to the substrate and being capable of achieving a highly purified factor VIII:C with a fold purification greater than 160,000 relative to plasma, said monoclonal antibody being bound to solid resin particles.

13. An immunoadsorbent according to Claim 12, wherein said resin comprises agarose.

14. An immunoadsorbent according to Claim 13, wherein said immunoadsorbent has a coupling density of 3 to 4g of monoclonal antibody per litre of agarose.

15. An immunoadsorbent according to any of Claims 12 to 14 wherein the antibody is an IgG.

16. An immunoadsorbent according to any of Claims 12-15, having the capability of adsorbing 90 to 100% of the VIII:RP present in plasma or commercial concentrate source applied thereto.

**Patentansprüche**

1. Verfahren zur Zubereitung des menschlichen Gerinnungsfaktor VIII-Aktivitätsproteins mit den Verfahrensschritten
   a) Adsorbieren eines VIII:C/VIII:AP-Komplexes von einem Plasma oder einer handelsüblichen Quelle auf ein festes Substrat, welches an einen für VIII:AP spezifischen monoklonalen Antikörper gebunden ist, und welches an VIII:AP gebunden bleibt , wenn es einer salinischen Lösung ausgesetzt wird, während es an das Substrat gebunden ist, wobei der monoklonale Antikörper, im Verfahrensschritt (d) weiter unten, in der Lage ist ein VIII:C-Produkt durch eine Vielfach-Reinigung von größer als 169.000 im Verhältnis zum Plasma zu erreichen;
   b) Auswaschen des VIII:C;
   c) Konzentrieren des im Verfahrensschritt b) durch Affinitäts-Chromatographie erhaltenen VIII:C;
   d) Rückgewinnung von hoch gereinigtem und konzentriertem VIII:C.

2. Verfahren nach Anspruch 1, wobei das sowohl in Schritt b) als auch c) benutzte Reinigungsmittel eine gepufferte Salzlösung ist.

3. Verfahren nach Anspruch 2, wobei die Salzlösung Calciumchlorid enthält.

4. Verfahren nach Anspruch 3, wobei die Konzentration der besagten, in den Verfahrensschritten b) und c) benutzten, Calciumchloridlösung zwischen 0,24 Mol und 0,5 Mol beträgt.

5. Verfahren nach den Ansprüchen 1 bis 4, wobei das besagte Substrat im Verfahrensschritt a) die Form von festen Substratpartikeln besitzt.

6. Verfahren nach Anspruch 5, wobei das besagte Substrat Agarose ist.

7. Verfahren nach einem der Ansprüche 1 bis 6, wobei Aminohexyl-Agarose in Schritt c) als Adsorbens eingesetzt wird.

8. Verfahren nach einem der Ansprüche 1 bis 7, wobei der verwendete monoklonale Antikörper ein IgG ist.

9. Verfahren nach Anspruch 8, wobei der verwendete monoklonale Antikörper das IgG einer Maus ist.

10. Verfahren zur Isolierung des menschlichen Gerinnungsfaktor VIII-Aktivitätsproteins von einem Plasma oder einem Konzentrat einschließlich des Verfahrensschritts des Durchleitens des besagten Plasmas oder Konzentrats durch eine chromatographische Säule, welche ein Harz-Adsorbens enthält, welches an einen monoklonalen Antikörper gebunden ist, wie in Anspruch 1 definiert, und das Auswaschen des VIII:C hieraus.

11. Verfahren nach Anspruch 10, wobei das besagte Adsorbens Agarose und das besagte Reinigungsmittel eine Salzlösung ist.

12. Ein verbessertes Immunadsorbens zur Isolierung und Reinigung des menschlichen Gerinnungsfaktors VIII:C von VIII:C/VIII:AP bestehend aus einem für spezifischen VIII:AP monoklonalen Antikörper, welcher an VIII:AP gebunden bleibt, wenn er einer salinischen Lösung ausgesetzt wird, während er an das Substrat gebunden ist und einen hoch gereinigten Faktor VIII:C durch eine Vielfach-Reinigung von mehr als 160.000 im Verhältnis zum Plasma erreicht, wobei der besagte monoklonale Antikörper an feste Harz-Partikel gebunden ist.

13. Immunadsorbens nach Anspruch 12, wobei das besagte Harz Agarose enthält.

14. Immunadsorbens nach Anspruch 13, wobei das besagte Immunadsorbens eine Bindungsdichte von 3 bis 4 g des monoklonalen Antikörpers pro Liter Agarose besitzt.

15. Immunadsorbens nach einem der Ansprüche 12 bis 14, wobei der Antikörper ein IgG (Immunglobulin G)ist.

16. Immunadsorbens nach einem der Ansprüche 12 bis 15, welches in der Lage ist, 90 bis 100% des im Plasma oder in sonstigem hierbei verwendeten Konzentrat enthaltenen VIII-AP zu adsorbieren.

## Revendications

1. Procédé de préparation de la protéine à activité procoagulante Facteur VIII humain, comprenant les étapes consistant à
   a) adsorber un complexe VIII:C/VIII:RP d'un plasma ou d'une source commerciale sur un substrat solide auquel est lié un anticorps monoclonal qui est spécifique à VIII:RP et qui reste complexé à VIII:RP quand il est soumis à une solution saline pendant qu'il est lié au substrat, l'anticorps monoclonal étant capable de fournir, dans l'étape (d) ci-dessous, un produit VIII:C avec un degré de purification de plus de 160 000 par rapport au plasma;
   b) à éluer le VIII:C;

c) à concentrer le VIII:C obtenu dans l'étape (b) par chromatographie d'affinité;
d) à recueillir VIII:C hautement purifié et concentré.

2. Procédé selon la revendication 1, dans lequel l'éluant utilisé dans chacune des étapes (b) et (c) est une solution de sels tamponnée.

3. Procédé selon la revendication 2, dans lequel la solution de sels contient du chlorure de calcium.

4. Procédé selon la revendication 3, dans lequel la concentration de ladite solution de chlorure de calcium utilisée dans les étapes (b) et (c) est comprise entre 0,24M et 0,5M.

5. Procédé selon l'une quelconque des revendications 1 à 4, dans lequel ledit substrat dans l'étape (a) est sous la forme de particules de substrat solides.

6. Procédé selon la revendication 5, dans lequel ledit substrat est de l'agarose.

7. Procédé selon l'une quelconque des revendications 1 à 6, dans lequel on utilise comme adsorbant dans l'étape (c) de l'aminobexylagarose.

8. Procédé selon l'une quelconque des revendications 1 à 7, dans lequel l'anticorps monoclonal utilisé est une IgG.

9. Procédé selon la revendication 8, dans lequel l'anticorps monoclonal utilisé est une IgG de souris.

10. Procédé de purificarion de la protéine à activité procoagulante Facteur VIII humain à partir de plasma ou de concentré, comprenant l'étape consistant à faire passer ledit plasma ou concentré dans une colonne chromatographique contenant une résine adsorbante à laquelle sont liés des anticorps monoclonaux tels que définis dans la revendication 1 et à en éluer le VIII:C.

11. Procédé selon la revendication 10, dans lequel ledit adsorbant est de l'agarose et ledit éluant est une solution d'un sel.

12. Immunoadsorbant amélioré pour l'isolement et la purification de VIII:C à partir de VIII:C/VIII:RP comprenant un anticorps monoclonal spécifique à VIII:RP, qui reste complexé à VIII:RP quand il est soumis à une solution saline pendant qu'il est lié au substrat et qui peut donner un facteur VIII:C ultrapurifié avec un degré de purification supérieur à 160 000 par rapport au plasma, ledit anticorps monoclonal étant lié à des particules de résine solides.

13. Immunoadsorbant selon la revendication 12, dans lequel ladite résine comprend de l'agarose.

14. Immunoadsorbant selon la revendication 13, qui a une densité de couplage de 3 à 4 g d'anticorps monoclonal par litre d'agarose.

15. Immunoadsorbant selon l'une quelconque des revendications 12 à 14, qui est une IgG.

16. Immunoadsorbant selon l'une quelconque des revendications 12 à 15, ayant la possibilité d'adsorber 90 à 100% du VIII:RP présent dans du plasma ou dans une source concentrée commerciale qui lui est appliqué.